# EUROPEAN PATENT SPECIFICATION

(11) **EP 3 569 283 B1**
(45) Date of publication and mention of the grant of the patent: **01.11.2023**
(21) Application number: 18173218.1
(22) Date of filing: 18.05.2018
(51) Int. Cl.: A61N 1/05, A61B 17/34

(54) **A LEAD PADDLE POSITIONING AND/OR DEPLOYMENT SYSTEM, A LEAD PADDLE AND A STYLET**
ELEKTRODEN-PADDLE-POSITIONIERUNGS- UND/ODER -EINSETZSYSTEM, EIN ELEKTRODEN-PADDLE UND EIN FÜHRUNGSSTAB
SYSTÈME DE POSITIONNEMENT ET/OU DE DÉPLOIEMENT DE PALETTE DE BRANCHEMENT, PALETTE DE BRANCHEMENT ET STYLET

(43) Date of publication of application: 20.11.2019
(73) Proprietor: ONWARD Medical N.V., 5611 ZX Eindhoven (NL)
(72) Inventor: Ganty, Damian, 5656 AE Eindhoven (NL); Delattre, Vincent, 5656 AE Eindhoven (NL)
(74) Representative: DTS Patent- und Rechtsanwälte Schnekenbühl und Partner mbB

(56) References cited:
- US-A1- 2010 179 562
- US-A1- 2012 271 315
- US-A1- 2013 268 041
- US-A1- 2015 151 114
- US-A1- 2015 224 326

## Description

The present invention relates to a lead paddle positioning and/or deployment system comprising at least a stylet and a lead paddle, a stylet and a lead paddle.

At the moment it seems that the existing technique and surgical procedures do not allow placing easily a lead paddle at the right and intended place into the epidural space, especially for long and wide arrays / paddles.

This technique might be helpful especially for "difficult cases" such as patients with scar tissues, fibrosis, calcification due to previous surgeries or lead replacement. The stylet would help to safely steer and push the paddle into the canal at the intended place.

Some single lumen stylet may already exist for the need insertion but there is also a trade-off between pushabilities / steerabilities and safety due to stylet material stiffness diameter limits.

Lead paddles are for example known from US 8,108,051 B2, US 2013/0096662 A1, US 2012/0006793 A1 and EP 3 013 411 A1.

A stylet system is for example known from US 6,309,401 B1 or from US 2003/0135253 A1, which relates to a neurostimulation lead stylet handle. US 2015/0224326 A1 shows a soft gripping surgical tool including one or more micro-channels for bending the tool towards or away from a surgical site.

It is therefore an object of the present invention to provide a lead paddle positioning and/or deployment system, a stylet for a lead paddle system and a lead paddle, in particular in that the axial stiffness and radial flexibility of a lead paddle is increased and a safe and accurate positioning of the lead paddle can be insured.

This object is solved according to the present invention by a lead paddle positioning and/or deployment system with the features of claim 1. The scope of the invention is defined by the appended claims. For better understanding of the invention, in the following disclosure multiple examples of a leads paddle positioning and/or deployment system are provided.

The present disclosure is based on a basic idea that by providing a guiding channel the form and stability of the lead paddle can already be influenced in terms of axial stiffness and radial flexibility. For easier deployment a stylet is provided, which allows maneuvering and handling of the whole system via a minimally invasive incision in connection with the implantation side.

In particular, the guiding channel may be U-shaped.

The guiding channel with its U-shaped form may have a round part and a closed part at its distal end and at its proximal two ends.

The two ends may allow introducing a guiding wire. The guiding wire may be the guiding wire of the stylet.

According to the invention the lead paddle comprises two guiding channels. By providing two guiding channels, more options for introducing the guiding wires of a stylet may be provided.

The guiding channels may extend over more then a half of the length of the length of the lead paddle, especially it is possible that the guiding channel extends over more then half or more then 80% of the length of the lead paddle.

In particular, it is possible that the guiding channel(s) is/are arranged along the outer edge region of the lead paddle. In particular, if the outer form of the lead paddle and the outer edge of the lead paddle already comprises an elongated form with a round tip, then with a U-shape the guiding channel is following the form of the outer edge of the lead paddle.

Furthermore, the guiding channels may be connected by a connection section, such that the overall form of the guiding channels with the connection section is forming a connected guiding channel structure, which is U-shaped. By this, the form of the guiding channel structure enhances the overall stability of the lead paddle and provides additional radial flexibility in connection with axial stiffness.

The guiding wire may be flexible and/or bendable. By this, the guiding wire can be inserted more easily into a guiding channel or a guiding channel structure and also follow the guiding channel or the guiding channel structure.

For example, the guiding wire can be inserted by sliding into the guiding channel structure, such that the stylet wire can be single and inserted / removed from one extremity of the guiding channels and exit from the other extremity of the guiding channels, the overall shape of the guiding wire is then forming a U-shape in the guiding channel structure.

According to the invention, the stylet comprises two guiding wires. By two guiding wires better steering of the lead paddle, when engaged with the stylet, is possible. Furthermore, it is possible that the stylet may have a fork-like form. By this, the engagement with the guiding channel and the guiding wire is easier possible. The guiding wires or the guiding wire may be dis-engageable with the stylet.

Furthermore, the stylet may comprise a U-shaped guiding-wire receiving portion. With such a U-shaped guiding wire receiving portion stability on the one hand and a better introduction of forces over moments and bending forces and flexibility can be provided.

The stylet may also comprise a handle, which may be connected to the U-shaped guiding-wire receiving portion. By means of the handle a better handling of the overall stylet may be provided. The handle may be made out of a stiff material such as metal of a re-enforced or stiff polymer / plastic.

Furthermore, it is possible that the guiding-wire receiving portion is curved. By this it is possible to allow easier deployment of the lead paddle via the incision during implantation.

The lead paddle positioning and/or deployment system may also be characterized in that the curvature of the guiding-wire receiving portion is less than the curvature of the curved connecting portion. This results in a form that the engaged stylet with the lead defines an angle of more than 90 degree, which is beneficial for implantation.

In particular, it is possible that the stylet has a proximal end, which defines with the distal end of the guiding wire an angle in the range of approx. 80° to 100°.

Moreover, it is possible that in the engaged state the lead paddle and the stylet have a curvature radius of the lead induced by the engagement with the guiding wires is approx. 90 to 110 mm, especially approx. 100mm, and/or that the angle between the lead paddle and the stylet, especially the U-shaped guiding-wire receiving portion, is chosen in the range of approx. 5° to 15°, especially at approx. 10°.

The guiding-wire receiving portion may be configured and formed such that in combination with a predetermined length of the guiding wire a portion of the guiding-wire receiving portion forms a mechanical stop, which reaches one edge of the lead paddle, when engaging the lead paddle and stylet. By this it can be prevented that the mechanical stop comes from the guiding wire, when inserting the guiding wire into the guiding channel of the lead paddle. This will help to avoid for example punctures of the lead paddle or the like.

Furthermore, the lead paddle positioning and/or deployment system comprises at least one lead body and a lead body connection portion, wherein the lead body connection portion is arranged on a flat side of the lead paddle with an offset to the edge of the proximal end of the lead paddle, especially centric with regard to the axial axis of the lead paddle, and wherein the lead body is connected to the lead paddle in the lead body connection portion. There may be one or more lead bodies. Possible embodiments may comprise for example two lead bodies. The lead bodies can be arranged centric with regard to the axial axis of the lead paddle and connected on the opposite side of the contact side of the lead paddle, i.e. the upper side of the lead paddle. The lead body connection portion may be arranged with an offset to the edge of the proximal end of the lead paddle, such that the lead paddle may be moved back and forth and also to the left and right and vice versa even after deployment in the spinal canal.

The lead paddle may especially have a U-shaped guiding channel. The guiding channel may be arranged along the outer edge region of the lead paddle.

The stylet material and diameter may be stiff enough to allow pushing and steering in the canal, i.e. the guiding wire.

The stylet material and diameter may be soft enough to be easily removable. For this, a coating such as Teflon might be very helpful and be used.

The U-shape main feature gives the "safety" compared to the single lumen stylet ball tip extremity.

The feature to have two stylet lumens on paddle sides instead of having only one at the center gives more steering and driving possibilities.

Furthermore, the above construction allows easy unclipping the handle from the stylet removal.

Also a ball tip at the stylet extremity is foreseen for a safe removal.

Furthermore, the stylet may be pre-bended with an adequate angle for insertion to the canal.

This allows more freedom of movement and better handling in the angle may be adjusted by the user insertion more easily.

Further details and advantages of the present disclosure shall now be disclosed in connection with the drawings.

It is shown in
- Fig. 1: a first embodiment of a lead paddle;
- Fig. 2: a lead paddle positioning and deployment system with the lead paddle of Fig. 1;
- Fig. 3: a schematical view of the deployment of the lead paddle by means of the lead paddle positioning and deployment system;
- Fig. 4: a further schematical view of the deployment of the lead paddle by means of the lead paddle positioning and deployment system;
- Fig. 5: a further embodiment of a lead paddle positioning and deployment system according to the present invention;
- Fig. 6: a further view of the lead paddle positioning and/or deployment system according to Fig. 5;
- Fig. 7: a further view of the lead paddle positioning and/or deployment system according to Fig. 5;
- Fig. 8: a further view of the lead paddle positioning and/or deployment system according to Fig. 5; and
- Fig. 9: a second embodiment of a lead paddle according to the present invention;

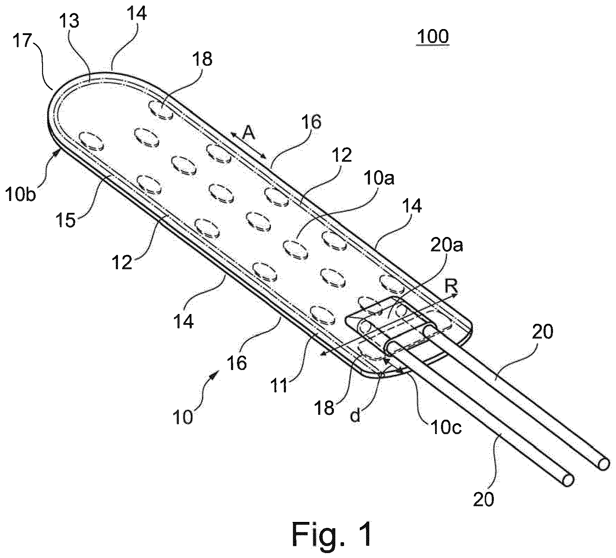

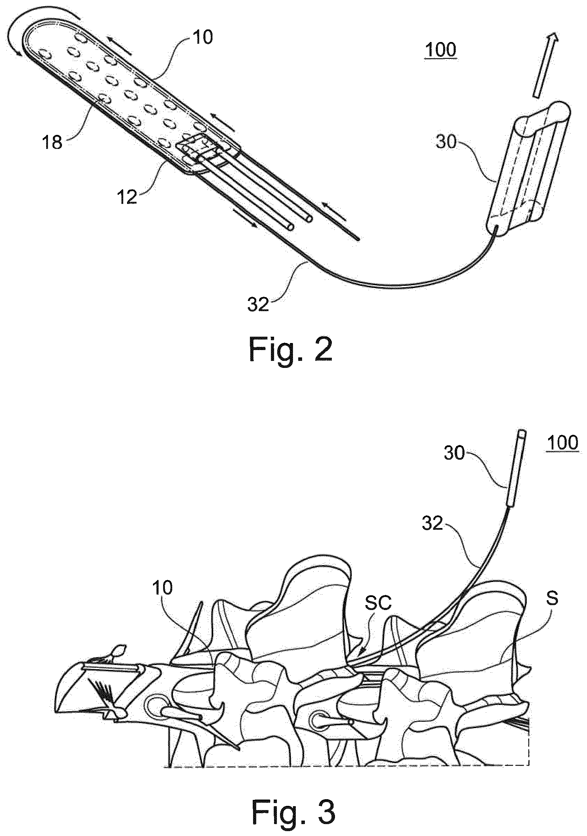

**Fig. 1** shows in a perspective view a possible embodiment of a lead paddle 10 of a lead paddle positioning and deployment system 100 (cf. **Fig. 2****).**

The lead paddle 10 comprises a lead paddle body 11 and comprises two guiding channels 12.

The two guiding channels 12 are embedded in the lead paddle body 11.

The lead paddle body 11 may be made of a medical grade material, such as a medical grade polymer. In particular, a medical grade silicone or the like may be used.

The guiding channels 12 extend over more then half of the length of the length of the lead paddle 10.

Especially, the guiding channels 12 extend over more then a half of 80% of the length of the lead paddle 10.

In particular, the guiding channels 12 are arranged along the outer edge region 14 of the lead paddle 10.

In particular, the guiding channels 12 are arranged parallel to the longitudinal edge 16 of the lead paddle 10.

The lead paddle 10 comprises a plurality of electrodes 18.

The electrodes 18 are embedded in the body of the lead paddle 10.

The shape of the electrodes 18 is rectangular.

The shape of all electrodes is more or less identical. Here the electrodes 18 are identical in their form.

Generally speaking, the form of one or more electrodes can be designed differently. In particular, they can be oval, round, square, diamond shape, trapezoidal or the like.

The arrangement of the electrodes 18 is also such that some electrodes are offset to each other.

The electrodes 18 of the lead paddle 10 are connected to lead bodies 20.

The lead bodies 20 are connected to a connection portion 20a on the upper side 10a of the lead paddle 10.

This connection portion 20a is on the opposite side of the contact side 10b of the lead paddle 10, that is configured and arranged to get in contact with the tissue to be stimulated, i.e. here the spinal cord of the patient.

Furthermore, the connection portion 20a is arranged centric with regard to the axial axis of the lead paddle 10. Moreover, the connection position 20a is positioned with an offset d to the edge of the proximal end 10c of the lead paddle 10.

As the connection portion 20a of the lead bodies 20 is not directly arranged at the outer edge of the lead paddle 10, the deployment and positioning of the lead paddle 10 is enhanced.

In particular, the offset d helps that by means of the lead bodies 20 the lead paddle 10 may be moved back and forth and also to the left and right and vice versa even after deployment in the spinal canal. As the lead bodies are arranged on the upper side with an offset to the edge, the proximal edge of the lead paddle is free and especially a movement in the proximal direction is not obstructed by the lead bodies.

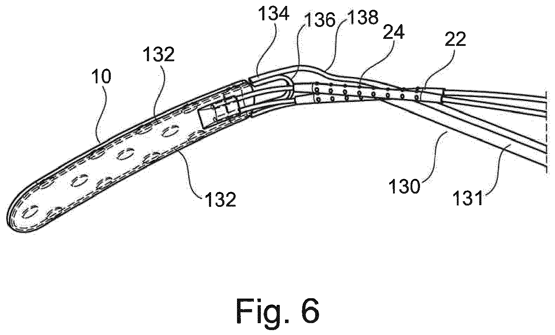

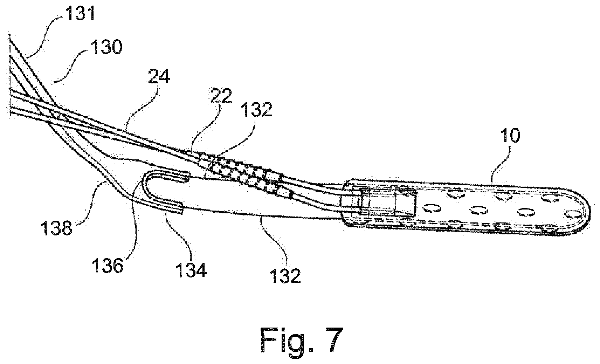

As can be seen in **Fig. 6** to **Fig. 8****,** the lead bodies 20 can be provided with anchoring sleeves 22.

The anchoring sleeves 22 are attached to the outer side of the lead body 20.

Furthermore, the anchoring sleeves 22 are provided with pins 24, which extend radially from the outer side of the anchoring sleeve 22.

The anchoring sleeves 22 are not shown in Fig. 1.

Moreover, the two guiding channels 12 are extended to the tip end 17 of the lead paddle body 11 with a connection section 13, such that the overall form of the guiding channels 12 with the connection section is forming a connected guiding channel structure 15, which is U-shaped.

The guiding channels 12 alone, but also the guiding channel structure 15 is configured and arranged such that guiding wires of a stylet may be received in the guiding channels 12 and thus stylet and lead paddle 10 may be engaged with each other for deployment and positioning.

The lead paddle body 11 is flexible by means of the chosen material, but also by its structure. However, the flexibility of the lead paddle body 11 is different in axial direction A compared to the radial direction R.

Stiffness in the axial direction A is provided by the electrodes 18 in connection with the guiding channels 12 and the U-shaped guiding channel structure 15.

Surprisingly, it was found in experimental setups and lead paddle deployments that at least one of the guiding channels 12 already and significantly enhances the axial stiffness, but does not affect the radial flexibility.

So, the paddle body 11 and thus the lead paddle 10 comprises axial stiffness in the direction A and radial flexibility in the direction R.

So, it is generally possible that even without a stylet and by means of the lead bodies 20 the lead paddle 10 can be inserted into the spinal channel. For such an insertion axial stiffness is necessary to avoid bending in axial direction, whereas (slight) bending in the radial direction is wanted to adapt to the anatomical structures at the implantation site in the spinal channel.

**Fig. 2** shows the embodiment of Fig. 1 of the lead paddle 10 in connection with the possible embodiment of a stylet 30 and a guiding wire 32.

The stylet 30 comprises at least one guiding wire 32 at its distal end, which is configured and arranged to be engaged and received by the guiding channel 12.

The guiding wire 32 is flexible and bendable.

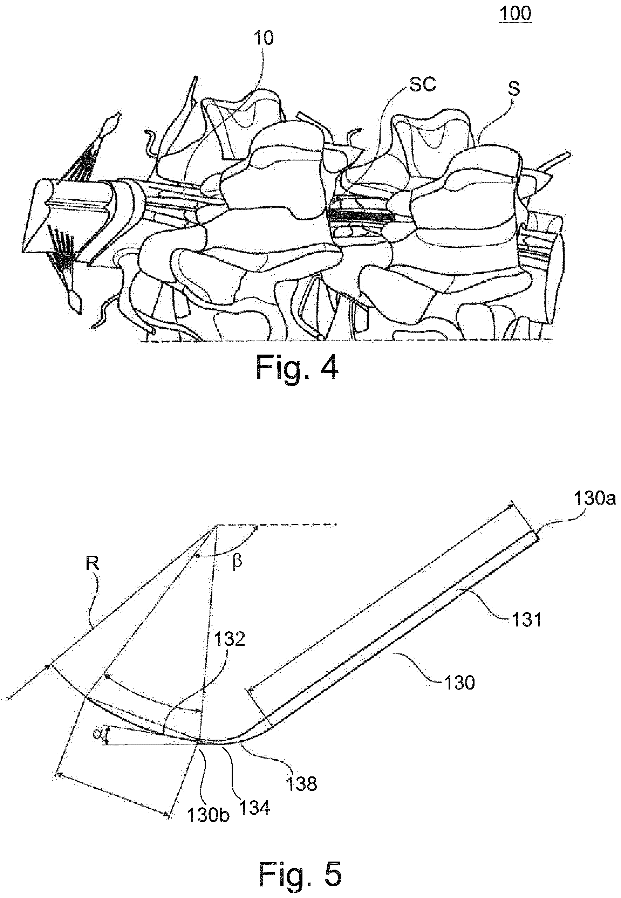

Reference is also made to Fig. 5-9, where according to the invention two guiding wires 32' are used (see also the embodiment of Fig. 5 to 8).

**Fig. 3** and **Fig. 4** show schematical views of the deployment of the lead paddle 10 by means of the lead paddle positioning and deployment system 100 into the spinal channel SC of the spinal column S.

The stylet 30 is during the deployment and positioning of the lead paddle 10 engaged with the lead paddle 10 by means of the lead wire 32.

The guiding wire 32 is in the situation shown in Fig. 3 positioned and engaged in and with the guiding channel structure 15 of the lead paddle 10.

For engagement, the flexibility and bendability of the guiding wire 32 ist used. By this, the guiding wire can be inserted more easily into the guiding channel structure 15 and also follow the guiding channel structure 15.

Here, the guiding wire can be inserted by sliding into the guiding channel structure, such that the stylet wire can be single and inserted / removed from one extremity of the guiding channels and exit from the other extremity of the guiding channels, the overall shape of the guiding wire is then forming a U-shape in the guiding channel structure.

So, the stylet may be moved towards the spinal column S and thereby the lead paddle 10 is pushed and moved forward in the spinal channel SC to the correct implantation position.

The positioning is shown in **Fig. 3****.**

After reaching the correct implantation position, the guiding wire 32 is disengaged with the guiding channel structure 15 of the lead paddle 10. Then the stylet 30 and the guiding wire 32 are removed and the lead paddle 10 is left in the spinal channel SC.

**Fig. 4** shows the correct positioned lead paddle 10 in the spinal channel SC.

**Fig. 5** shows a further embodiment of a stylet 130 of/for the lead paddle positioning and deployment system 100.

Each reference number of the stylet 30 according to Fig. 2 and Fig. 3 is in the embodiment shown in **Fig. 5** to **Fig. 8** increased by the value100 for similar or identical features of the stylet 30 according to Fig. 2 and Fig. 3.

The stylet 130 comprises each and every structural and functional feature as the stylet 30 according to Fig. 2 and Fig. 3. It can be used similar and/or identical like the stylet 30 as described above in connection with Fig. 2 and Fig. 3.

However, the stylet 130 as shown **Fig. 5** to **Fig. 8** comprises the following differences:
The stylet 130 has a handle 131.

The handle 131 may be made of a stiff material, like a stiff polymer or even a metal.

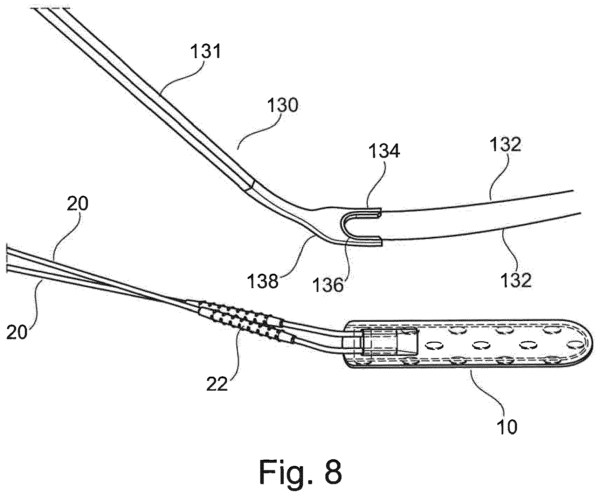

In **Fig. 5** to **Fig. 8****,** it becomes clear that the stylet 130, which is suitable for the embodiment of the lead paddle 10 or 10', has a fork like form (cf. e.g. Fig. 8).

Furthermore, the stylet 130 comprises a U-shaped guiding wire receiving portion 134.

The handle 131 is connected to the U-shaped guiding-wire receiving portion 134.

The guiding wire 132 itself may also be U-shaped and being received in a U-shaped receiving recess 136 within the U-shaped guiding wire receiving portion 134.

As the guiding wire 132 is flexible and bendable, the U-shaped form of the guiding wire 132 is achieved by inserting the guiding wire 132 into the receiving recess 136.

As the guiding wire 132 has two free ends, the stylet 130 comprises by this arrangement two guiding wires 132 provided by the free ends of the guiding wire 132 extending out of the U-shaped receiving recess 136.

The U-shaped guiding-wire receiving portion 134 and the handle 131 are connected to each other with a curved connecting portion 138.

The guiding wire receiving portion 134 is curved.

Furthermore, the curvature of the guiding wire receiving portion 134 is less than the curvature of the curved connecting portion 138.

Fig. 5 also shows details on angles and radius of the design of the stylet 130.

The stylet 130 has a proximal end 130a and a distal end 130b.

The proximal end 130a defines with the distal end 130b, but also with the distal end of the guiding wire 132 an angle β in the range of approx. 80° to 100°.

The guiding wire receiving portion 134 is configured and formed such that in combination with a predetermined length of the guiding wire 132 a portion of the guiding-wire receiving portion 134 forms a mechanical stop, which reaches one edge of the lead paddle 10, when engaging the lead paddle 10 and stylet 130.

In the engaged state of the lead paddle 10 and the stylet 130, the curvature radius R of the lead paddle 10 induced by the engagement with the guiding wires 132 is approx. 90 to 110 mm, here in the shown embodiment of Fig. 5 it is 100mm.

The angle α between the lead paddle 10 and the stylet 130, here with the U-shaped guiding-wire receiving portion 134, is chosen in the range of approx. 5° to 15°, here at 10°.

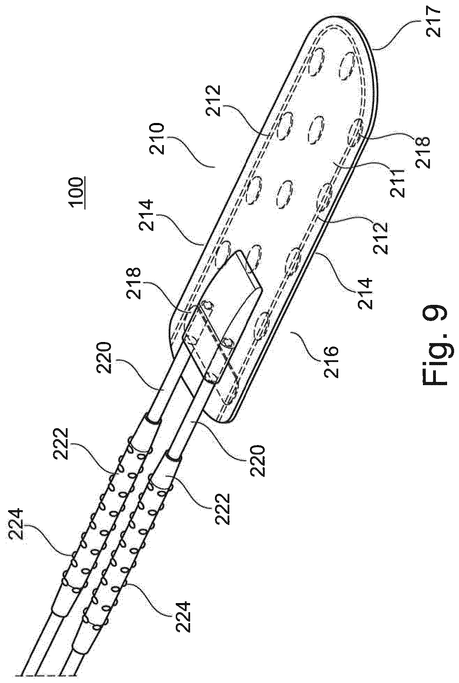

**Fig. 9** shows a further embodiment of a lead paddle 210 for the lead paddle positioning and deployment system 100.

Each reference number of the lead paddle 10 according to Fig. 1 is in the embodiment shown in Fig. 9 increased by the value 200 for similar of identical features of the lead paddle 10 according to Fig. 1.

The lead paddle 210 comprises each and every structural and functional feature as the lead paddle 10 according to Fig. 1.

However, the lead paddle 210 comprises the following differences:
According to the invention, the lead paddle 210 comprises two separate guiding channels 212.

The guiding channels 212 are not connected to each other.

The guiding channels 212 are also parallel to each other and completely straight.

On each lead body 220 have one anchoring sleeve 222 with the pins 224 is arranged.

### References

- 10: lead paddle
- 10a: upper side
- 10b: contact side
- 10c: proximal end
- 11: lead paddle body
- 12: guiding channel
- 13: connecting portion
- 14: outer edge region
- 15: guiding channel structure
- 16: longitudinal edge
- 17: tip end
- 18: electrodes
- 20: lead body
- 20a: connection portion
- 22: anchoring sleeves
- 24: pins
- 30: stylet
- 32: guiding wire
- 100: lead paddle positioning and deployment system

- 130: stylet
- 130a: proximal end of the stylet
- 130b: distal end of the stylet
- 131: handle
- 132: guiding wire
- 134: U-shaped guiding wire receiving portion
- 136: U-shaped receiving recess
- 138: curved connecting portion

- 210: lead paddle
- 211: lead paddle body
- 212: guiding channel
- 214: outer edge region
- 216: longitudinal edge
- 217: tip end
- 218: electrodes
- 220: lead body
- 222: anchoring sleeves
- 224: pins

- d: offset

- R: radius
- S: spinal column
- SC: spinal channel

- α: angle
- β: angle

## Claims

1. A lead paddle positioning and/or deployment system (100) comprising at least a stylet (30; 130) and a lead paddle (10; 210), the lead paddle (10; 210) being configured for stimulating a tissue, in particular the spinal cord, of a patient, wherein the lead paddle (10; 210) comprises two guiding channels (12; 212) and wherein the stylet (30; 130) comprises two guiding wires (32; 132) at its distal end, which are configured and arranged to be engaged and/or to be at least partially received in the guiding channels (12; 212).

2. The lead paddle positioning and/or deployment system (100) according to claim 1, wherein the guiding channels (12; 212) extend over more than the half of the length of the length of the lead paddle (10; 210), especially wherein the guiding channels (12; 212) extend over more than the half of 80% of the length of the lead paddle (10; 210).

3. The lead paddle positioning and/or deployment system (100) according to claim 1 or clam 2, wherein the guiding channels (12; 212) are arranged along the outer edge region (14; 214) of the lead paddle (10; 210).

4. The lead paddle positioning and/or deployment system (100) according to one of the claims 1 to 3, wherein the guiding channels (12) are connected by a connection section (13), such that the overall form of the guiding channels (12) with the connection section (13) is forming a connected guiding channel structure (15), which is U-shaped.

5. The lead paddle positioning and/or deployment system (100) according to one of the preceding claims, wherein the guiding wires (32, 132) flexible and/or bendable.

6. The lead paddle positioning and/or deployment system (100) according to one of the preceding claims, wherein the stylet (130) has a fork-like form.

7. The lead paddle positioning and/or deployment system (100) according to one of the preceding claims, wherein the stylet (130) comprises a U-shaped guiding-wire receiving portion (134).

8. The lead paddle positioning and/or deployment system (100) according to claim 7, wherein the stylet (130) comprises a handle (131), which is connected to the U-shaped guiding-wire receiving portion (134).

9. The lead paddle positioning and/or deployment system (100) according to claim 7 or claim 8, wherein the U-shaped guiding-wire receiving portion (134) and the handle are connected to each other with a curved connecting portion.

10. The lead paddle positioning and/or deployment system (100) according to one of claims 7 to 9, wherein the guiding-wire receiving portion (134) is curved.

11. The lead paddle positioning and/or deployment system (100) according to claim 9 and claim 10, wherein the curvature of the guiding-wire receiving portion (134) is less than the curvature of the curved connecting portion.

12. The lead paddle positioning and/or deployment system (100) according to one of claims 7 to 11, wherein the stylet (130) has a proximal end, which defines with the distal end of the guiding wire (132) an angle (β) in the range of approx. 80° to 100°.

13. The lead paddle positioning and/or deployment system (100) according to one of claims 8 to 12, wherein the guiding-wire receiving portion (134) is configured and formed such that in combination with a predetermined length of the guiding wire (132) a portion of the guiding-wire receiving portion (134) forms a mechanical stop, which reaches one edge of the lead paddle (10; 210), when engaging the lead paddle (10; 210) and stylet (130).

14. The lead paddle positioning and/or deployment system (100) according to one of the preceding claims, wherein in the engaged state of the lead paddle (10; 210) and the stylet (130) the curvature radius (R) of the lead induced by the engagement with the guiding wires (132) is approx. 90 to 110 mm, especially approx. 100mm, and/or that the angle (α) between the lead paddle (10; 210) and the stylet (130), especially the U-shaped guiding-wire receiving portion (134), is chosen in the range of approx. 5° to 15°, especially at approx. 10°.

15. The lead paddle positioning and/or deployment system (100) according to one of the preceding claims, wherein the lead paddle positioning and/or deployment system (100) comprises at least one lead body (20) and a lead body connection portion (20a), wherein the lead body connection portion (20a) is arranged on a flat side of the lead paddle (10) with an offset (d) to the edge of the proximal end (10c) of the lead paddle (10), especially centric with regard to the axial axis of the lead paddle (10), and wherein the lead body (20) is connected to the lead paddle (10) in the lead body connection portion (20a).

## Patentansprüche

1. Elektroden-Paddle-Positionierungs- und/oder -Einsetzsystem (100) mit mindestens einem Führungsstab (30; 130) und einem Elektroden-Paddle (10; 210), wobei das Elektroden-Paddle (10; 210) dafür ausgelegt ist, ein Gewebe, insbesondere das Rückenmark, eines Patienten zu stimulieren, wobei das Elektroden-Paddle (10; 210) zwei Führungskanäle (12; 212) aufweist und der Führungsstab (30; 130) zwei Führungsdrähte (32; 132) an seinem distalen Ende aufweist, die dafür ausgelegt und eingerichtet sind, in den Führungskanälen (12; 212) in Eingriff und/oder mindestens zum Teil aufgenommen zu sein.

2. Elektroden-Paddle-Positionierungs- und/oder -Einsetzsystem (100) nach Anspruch 1, wobei sich die Führungskanäle (12; 212) über mehr als die Hälfte der Länge der Länge des Elektroden-Paddles (10; 210) erstrecken, insbesondere wobei sich die Führungskanäle (12; 212) über mehr als die Hälfte von 80% der Länge des Elektroden-Paddles (10; 210) erstrecken.

3. Elektroden-Paddle-Positionierungs- und/oder -Einsetzsystem (100) nach Anspruch 1 oder Anspruch 2, wobei die Führungskanäle (12; 212) entlang des Außenrandbereichs (14; 214) des Elektroden-Paddles (10; 210) angeordnet sind.

4. Elektroden-Paddle-Positionierungs- und/oder -Einsetzsystem (100) nach einem der Ansprüche 1 bis 3, wobei die Führungskanäle (12) über einen Verbindungsabschnitt (13) so verbunden sind, dass die Gesamtform der Führungskanäle (12) mit dem Verbindungsabschnitt (13) eine verbundene Führungskanalstruktur (15) bildet, die U-förmig ist.

5. Elektroden-Paddle-Positionierungs- und/oder -Einsetzsystem (100) nach einem der vorhergehenden Ansprüche, wobei die Führungsdrähte (32, 132) flexibel und/oder biegbar sind.

6. Elektroden-Paddle-Positionierungs- und/oder -Einsetzsystem (100) nach einem der vorhergehenden Ansprüche, wobei der Führungsstab (130) eine gabelartige Form hat.

7. Elektroden-Paddle-Positionierungs- und/oder -Einsetzsystem (100) nach einem der vorhergehenden Ansprüche, wobei der Führungsstab (130) einen U-förmigen Führungsdraht-Aufnahmeabschnitt (134) aufweist.

8. Elektroden-Paddle-Positionierungs- und/oder -Einsetzsystem (100) nach Anspruch 7, wobei der Führungsstab (130) ein Griffteil (131) aufweist, das mit dem U-förmigen Führungsdraht-Aufnahmeabschnitt (134) verbunden ist.

9. Elektroden-Paddle-Positionierungs- und/oder -Einsetzsystem (100) nach Anspruch 7 oder Anspruch 8, wobei der U-förmige Führungsdraht-Aufnahmeabschnitt (134) und das Griffteil mit einem gekrümmten Verbindungsabschnitt miteinander verbunden sind.

10. Elektroden-Paddle-Positionierungs- und/oder -Einsetzsystem (100) nach einem der Ansprüche 7 bis 9, wobei der Führungsdraht-Aufnahmeabschnitt (134) gekrümmt ist.

11. Elektroden-Paddle-Positionierungs- und/oder -Einsetzsystem (100) nach Anspruch 9 und Anspruch 10, wobei die Krümmung des Führungsdraht-Aufnahmeabschnitts (134) schwächer ist als die Krümmung des gekrümmten Verbindungsabschnitts.

12. Elektroden-Paddle-Positionierungs- und/oder -Einsetzsystem (100) nach einem der Ansprüche 7 bis 11, wobei der Führungsstab (130) ein proximales Ende hat, das mit dem distalen Ende des Führungsdrahts (132) einen Winkel (β) im Bereich von ca. 80° bis 100° definiert.

13. Elektroden-Paddle-Positionierungs- und/oder -Einsetzsystem (100) nach einem der Ansprüche 8 bis 12, wobei der Führungsdraht-Aufnahmeabschnitt (134) so ausgelegt und gebildet ist, dass in Kombination mit einer vorbestimmten Länge des Führungsdrahts (132) ein Abschnitt des Führungsdraht-Aufnahmeabschnitts (134) einen mechanischen Anschlag bildet, der einen Rand des Elektroden-Paddles (10; 210) erreicht, wenn er am Elektroden-Paddle (10; 210) und am Führungsstab (130) angreift.

14. Elektroden-Paddle-Positionierungs- und/oder -Einsetzsystem (100) nach einem der vorhergehenden Ansprüche, wobei im Eingriffszustand des Elektroden-Paddles (10; 210) und des Führungsstabs (130) der Krümmungsradius (R) der Elektrode, der durch den Eingriff mit den Führungsdrähten (132) bewirkt wird, ca. 90 bis 110 mm, insbesondere ca. 100 mm beträgt, und/oder dass der Winkel (α) zwischen dem Elektroden-Paddle (10; 210) und dem Führungsstab (130), insbesondere dem U-förmigen Führungsdraht-Aufnahmeabschnitt (134), im Bereich von ca. 5° bis 15°, insbesondere bei ca. 10°, gewählt wird.

15. Elektroden-Paddle-Positionierungs- und/oder -Einsetzsystem (100) nach einem der vorhergehenden Ansprüche, wobei das Elektroden-Paddle-Positionierungs- und/oder -Einsetzsystem (100) mindestens einen Elektrodenkörper (20) und einen Elektrodenkörper-Verbindungsabschnitt (20a) aufweist, wobei der Elektrodenkörper-Verbindungsabschnitt (20a) an einer Flachseite des Elektroden-Paddles (10) mit einem Versatz (d) zum Rand des proximalen Endes (10c) des Elektroden-Paddles (10) angeordnet ist, insbesondere zentrisch mit Bezug auf die axiale Achse des Elektroden-Paddles (10), und wobei der Elektrodenkörper (20) mit dem Elektroden-Paddle (10) in dem Elektrodenkörper-Verbindungsabschnitt (20a) verbunden ist.

## Revendications

1. Système de positionnement et/ou de déploiement de palette de branchement (100) comprenant au moins un stylet (30 ; 130) et une palette de branchement (10 ; 210), la palette de branchement (10 ; 210) étant configurée pour stimuler un tissu, notamment la moelle épinière, d'un patient, dans lequel la palette de branchement (10 ; 210) comprend deux canaux de guidage (12 ; 212) et dans lequel le stylet (30 ; 130) comprend deux câbles de guidage (32 ; 132) au niveau de son extrémité distale qui sont configurés et agencés pour être emboîtés et/ou pour être au moins en partie reçus dans les canaux de guidage (12 ; 212).

2. Système de positionnement et/ou de déploiement de palette de branchement (100) selon la revendication 1, dans lequel les canaux de guidage (12 ; 212) s'étendent sur plus de la moitié de la longueur de la longueur de la palette de branchement (10 ; 210), dans lequel les canaux de guidage (12 ; 212) s'étendent en particulier sur plus de la moitié de 80 % de la longueur de la palette de branchement (10 ; 210) .

3. Système de positionnement et/ou de déploiement de palette de branchement (100) selon la revendication 1 ou 2, dans lequel les canaux de guidage (12 ; 212) sont agencés le long de la région d'arête extérieure (14 ; 214) de la palette de branchement (10 ; 210).

4. Système de positionnement et/ou de déploiement de palette de branchement (100) selon l'une quelconque des revendications 1 à 3, dans lequel les canaux de guidage (12) sont reliés par une section de connexion (13), de telle sorte que la forme d'ensemble des canaux de guidage (12) avec la section de connexion (13) forme une structure de canal de guidage (15) connectée en forme de U.

5. Système de positionnement et/ou de déploiement de palette de branchement (100) selon l'une quelconque des revendications précédentes, dans lequel les câbles de guidage (32, 132) sont flexibles et/ou cintrables.

6. Système de positionnement et/ou de déploiement de palette de branchement (100) selon l'une quelconque des revendications précédentes, dans lequel le stylet (130) est en forme de fourche.

7. Système de positionnement et/ou de déploiement de palette de branchement (100) selon l'une quelconque des revendications précédentes, dans lequel le stylet (130) comprend une partie de réception de câble de guidage en forme de U (134).

8. Système de positionnement et/ou de déploiement de palette de branchement (100) selon la revendication 7, dans lequel le stylet (130) comprend une poignée (131) qui est reliée à la partie de réception de câble de guidage en forme de U (134).

9. Système de positionnement et/ou de déploiement de palette de branchement (100) selon la revendication 7 ou la revendication 8, dans lequel la partie de réception de câble de guidage en forme de U (134) et la poignée sont reliées entre elles au moyen d'une partie de connexion incurvée.

10. Système de positionnement et/ou de déploiement de palette de branchement (100) selon l'une quelconque des revendications 7 à 9, dans lequel la partie de réception de câble de guidage (134) est incurvée.

11. Système de positionnement et/ou de déploiement de palette de branchement (100) selon la revendication 9 et la revendication 10, dans lequel la courbure de la partie de réception de câble de guidage (134) est inférieure à la courbure de la partie de connexion incurvée.

12. Système de positionnement et/ou de déploiement de palette de branchement (100) selon l'une quelconque des revendications 7 à 11, dans lequel le stylet (130) a une extrémité proximale qui définit avec l'extrémité distale du câble de guidage (132) un angle (β) compris dans la plage d'approx. 80° à 100°.

13. Système de positionnement et/ou de déploiement de palette de branchement (100) selon l'une quelconque des revendications 8 à 12, dans lequel la partie de réception de câble de guidage (134) est configurée et formée de telle sorte qu'en combinaison avec une longueur prédéterminée du câble de guidage (132), une partie de la partie de réception de câble de guidage (134) forme une butée mécanique qui atteint une arête de la palette de branchement (10 ; 210) lors de la mise en prise de la palette de branchement (10 ; 210) et du stylet (130).

14. Système de positionnement et/ou de déploiement de palette de branchement (100) selon l'une quelconque des revendications précédentes, dans lequel dans l'état mis en prise de la palette de branchement (10 ; 210) et du stylet (130), le rayon de courbure (R) du branchement induit par la mise en prise avec les câbles de guidage (132) est d'approx. 90 à 110 mm, en particulier d'approx. 100 mm, et/ou que l'angle (α) entre la palette de branchement (10 ; 210) et le stylet (130), en particulier la partie de réception de câble de guidage en forme de U (134), est choisie dans la plage d'approx. 5° à 15°, en particulier d'approx. 10°.

15. Système de positionnement et/ou de déploiement de palette de branchement (100) selon l'une quelconque des revendications précédentes, dans lequel le système de positionnement et/ou de déploiement de palette de branchement (100) comprend au moins un corps de branchement (20) et une partie de connexion de corps de branchement (20a), dans lequel la partie de connexion de corps de branchement (20a) est agencée sur un côté plat de la palette de branchement (10) avec un décalage (d) par rapport à l'arête de l'extrémité proximale (10c) de la palette de branchement (10), en particulier de façon centrée par rapport à l'axe axial de la palette de branchement (10) et dans lequel le corps de branchement (20) est connecté à la palette de branchement (10) dans la partie de connexion de corps de branchement (20a).
